Europäisches Patentamt

European Patent Office

Office européen des brevets

(11) Publication number: **0 387 026**
**A2**

# EUROPEAN PATENT APPLICATION

(21) Application number: 90302417.2

(22) Date of filing: 07.03.90

(51) Int. Cl.5 **C12M 1/40, //G01N33/543, G01N27/327, G01N27/416, C12Q1/54**

(30) Priority: **10.03.89 GB 8905507**

(43) Date of publication of application:
**12.09.90 Bulletin 90/37**

(84) Designated Contracting States:
**DE FR IT NL**

(71) Applicant: **PLESSEY OVERSEAS LIMITED**
**Vicarage Lane**
**Ilford Essex IG1 4AQ(GB)**

Applicant: **Lowe, Christopher Robin**
**The Limes, Hempstead**
**Saffron Walden, Essex, CB10 2PW(GB)**

(72) Inventor: **Sethi, Rajinder Singh**
**68 Westone Avenue**
**Northampton, NN3 3JQ(GB)**
Inventor: **Yonhin, Foo Yueh Yin**
**7 Izaak Walton Way, Chesterton**
**Cambridge, CB4 1TY(GB)**
Inventor: **Lowe, Christopher Robin**
**The Limes, Hempstead**
**Saffron Walden, Essex, CB10 2PW(GB)**

(74) Representative: **Pritchard, Evan**
**Intellectual Property Department The**
**Plessey Company plc Vicarage Lane**
**Ilford Essex IG1 4AQ(GB)**

(54) **Biosensor device.**

(57) A biosensor device comprises two spaced pairs 6 of electrodes supported on a surface of electrical insulation material on a silicon substrate 2, one of said electrode pairs 6 including a body of an immobilized reagent material incorporating an active biological catalyst which body is positioned between the electrodes of said pair to constitute a working electrode structure.

This can form an electrochemical cell where the reaction specific property of a biological catalyst can be utilised to permit analysis for a single component in a mixture.

Fig.1

## BIOSENSOR DEVICE

This invention relates to a biosensor device. It relates particularly to an amperometric biosensor which is capable of working by means of an electrochemical process and in which an immobilized biocatalyst material can be used.

In clinical analysis, it is important to be able to make selective determinations of certain organic compounds when these are present in biological fluids. The use of an electrochemical sensor employing an immobilized biocatalyst has been shown to have particular advantages in requiring only a simple test procedure and a short reaction time. An enzyme sensor can have good selectivity for a biological substrate and this can allow analysis for a single compound in a complicated mixture without a need for first separating the individual mixture components.

An amperometric biosensor can be capable of combining the specificity and selectivity of the enzyme catalyst with the analytical power of electrochemistry. As an example, the oxidation of glucose in the presence of the flavin-containing enzyme glucose oxidase (GOD, EC 1.1.3.4) proceeds according to the following scheme:

glucose + GOD (FAD +) -> gluconolactone + GOD (FADH$_2$) O$_2$ + GOD (FADH$_2$) -> H$_2$O$_2$ + GOD (FAD +)

where FAD + and FADH$_2$ represent the oxidised and reduced forms of flavin adenine dinucleotide respectively.

A convenient way to observe the progress of this reaction would be to monitor the generation of hydrogen peroxide. The mode of operation of the biosensor can be based on an electrochemical oxidation of the hydrogen peroxide reaction product as it is formed.

Rapid quantitative determination of glucose is necessary not only in analytical clinical laboratories but also for on-line supervision of diabetic patients. In the microbiological and food industries the use of a glucose sensor can be important for process control.

Galactose provides an alternative carbohydrate energy source and it improves the homeostatic regulation of glucose in the premature infant. Owing to its potentially toxic effects, accurate methods for monitoring its concentration in the serum in the 0.24mmol per litre are required. The present multianalyte amperometric microbiosensor is likely to be useful in these applications.

The microfabricated device also offers potential for the measurement of many substances of clinical or industrial importance when their complementary enzymes are immobilized onto the sensor electrode surface.

Thus, for example, a range of oxidases responding to a variety of substances such as L-amino acids, sugars, carboxylic acids, steroids and nitrogen heterocycles could be immobilized. Further other redox systems based on nicotinamide adenine dinucleotide (phosphate) (NAD)(P)-dependent oxidoreductases, pyrroloquinoline quinone (PQQ), tetrapyrrole and metal containing proteins could be exploited especially in combination with appropriate redox mediators to facilitate electron exchange between the redox protein and the electrode. Similarly, the microdevice could be used in conjunction with biological recognition systems other than single enzymes in order to extend the range of analytes amenable to assay. Thus, for example, multiple-enzyme sequences, subcellular organelles, whole microbial or other cells, immunoamperometric enzyme systems and receptor proteins could also be immobilized in close proximity to the sensor. Furthermore, applications of the device are not restricted to clinical diagnostics. Microamperometric sensors will find application in bioprocess control of fermenters and bioreactors, in veterinary, agricultural, horticultural analysis, in the monitoring of pollutants and toxic wastes and in security areas.

In some respects, the present application can be considered as a modification of the invention described in copending patent application No. GB 2204408A. This published application discloses a biosensor device comprising a pair of substantially parallel electrical conductors supported on a surface of electrical insulation material, and extending thereover an immobilized reagent material whereby, on exposing the reagent material to an analyte, variations in an electrical or thermal characteristic of the analyte/reagent may be measured.

The present invention was devised to provide a biosensor device which would enable the reaction specific property of an enzyme catalyst to be fully utilised.

According to the present invention, there is provided a biosensor device comprising two spaced pairs of electrodes supported on a surface of electrical insulation material on a silicon substrate, one of said electrode pairs including a body of an immobilized reagent material incorporating an active biological catalyst which body is positioned between the electrodes of said pair to constitute a working electrode structure.

Preferably, the support surface additionally carries a third electrode pair to constitute a reference electrode structure. The second electrode pair may include a body of an immobilized reagent material. In a different embodiment, the second electrode pair includes a body of an immobilized reagent material incorporating a second active biological catalyst.

The support surface may additionally carry a further electrode having a surface area greater than that of said one electrode pair, the further electrode forming a counter electrode structure.

The electrodes may be formed as thin film layers by an integrated circuit fabrication technique.

The electrodes may be formed of a noble metal such as gold or platinum.

The invention also comprises a method of fabricating a biosensor device, in which the materials for forming an integral reference electrode are deposited by an electrolytic process. The biological catalyst material may be deposited by an electrolytic process. Preferably, the biological catalyst deposition is effected simultaneously with deposition of a polymer support film. The surface for the proposed biological catalyst deposition may be initially masked with a non-electrically conductive material having a window formed therein, such that the deposit will only occur on a selected portion of the substrate. After deposition of a first biological catalyst material, the substrate may be masked again and a second window formed in the masking layer, the second window may then serve for deposition of a second biological catalyst on a different portion of the substrate.

By way of example, a particular embodiment of the invention will now be described with reference to the accompanying drawings, in which:

Figure 1 shows a biosensor device with one pattern for the electrical conductor arrangement on a substrate of electrical insulation material,

Figure 2 is a flow chart illustrating somewhat schematically the process stages required in the construction of the biosensor device, and,

Figures 3, 4 and 5 are graphs illustrating concentration measurements made with the sensor.

The construction of the biosensor device of the invention begins with the fabrication of a pattern of electrical conductors on a silicon chip and this is effected essentially by the method disclosed in the aforementioned copending patent application.

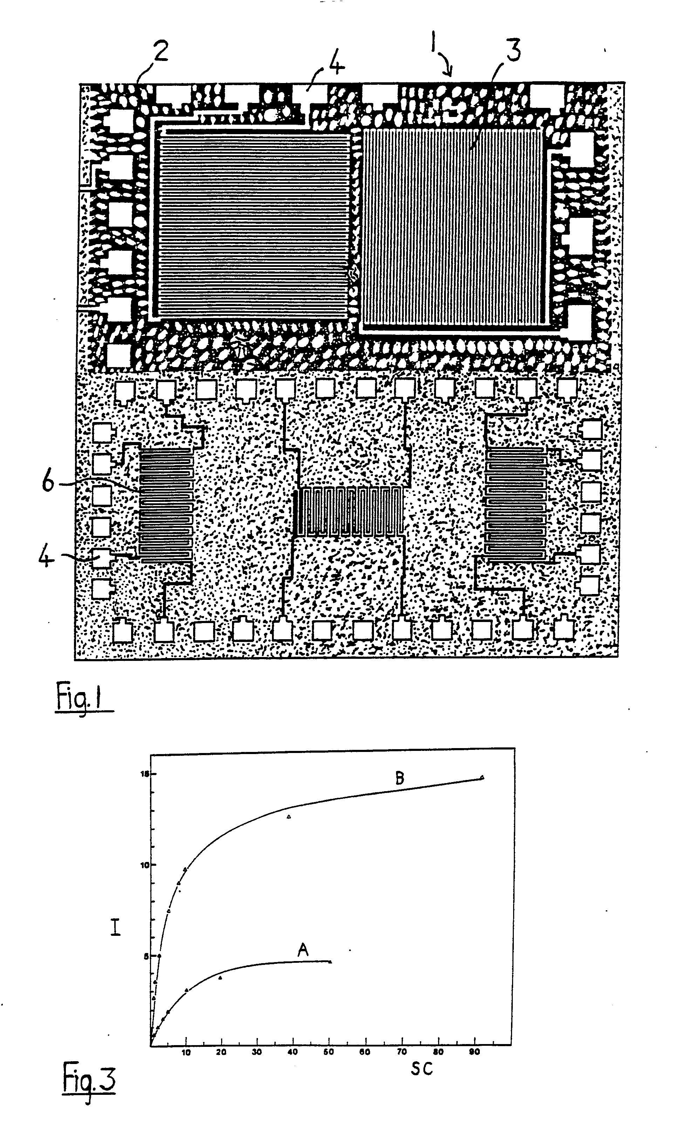

As depicted in Figure 1, the biosensor device 1 comprises a silicon chip 2 which measures 2.5 millimetres square and which is provided with a surface layer of silicon dioxide which supports various electrode areas. In the embodiment of Figure 1, the upper half of the drawing carries two patterns each of which has a folded electrode 3 structure which terminates at an electrode contact pad 4 located on the periphery of the chip.

The two folded electrode 3 patterns each have a comparatively large surface area as compared with further electrode areas on the chip. They can be used separately or together by connecting them in series to form a counter electrode structure for the proposed electrochemical cell.

The lower half of the drawing shows three electrode pair 6 structures which are arranged with a central pair in a generally horizontal attitude and the two outer pairs in a vertical attitude. The ends of the electrode pair 6 structures are brought out to contact pads 4 arranged near the chip edges.

The two outer pairs of electrodes are intended to be used to form sensing or working electrodes for the cell whilst the inner pair of electrodes is used to form a reference electrode for the cell. The electrodes of the biosensor device 1 may be readily joined to other circuitry for electrochemical measurements by attaching suitable connectors to the contact pads 4 at the edges of the chip 2.

The construction of the biosensor device begins with the provision of a silicon wafer which is put through various treatment stages which will generally be familiar to those engaged in semiconductor device production.

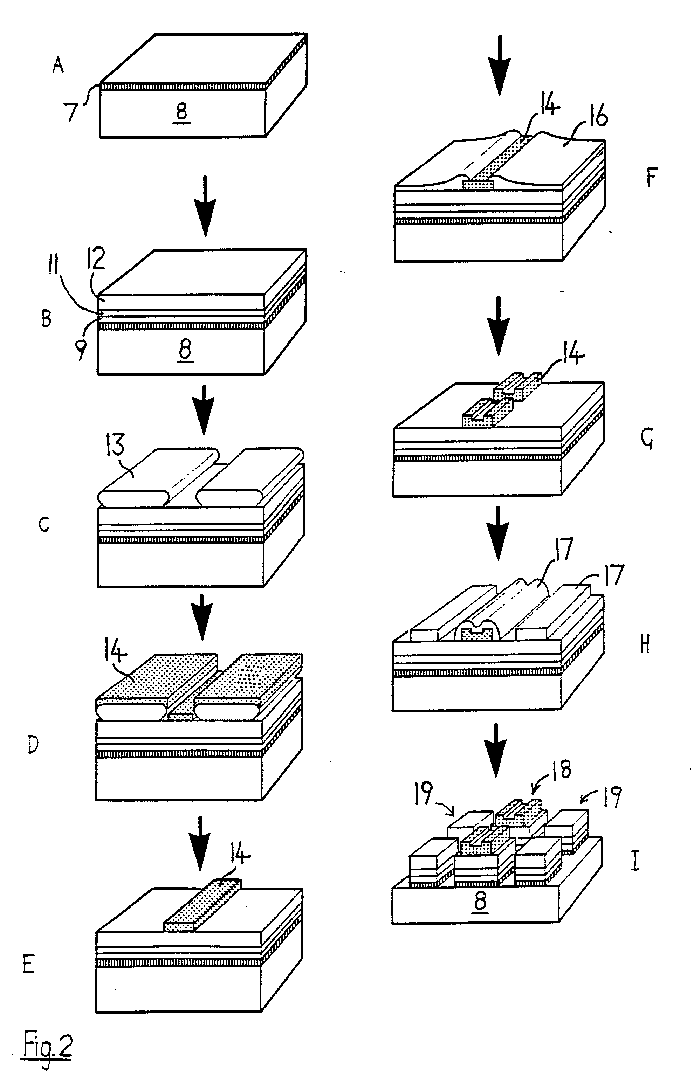

The first step is a surface thermal oxidation of the wafer material which produces a layer 7 of silicon dioxide 550nm in thickness on the silicon wafer 8 (Figure 2A).

A metallisation stage is then carried out by sputter deposition and electron beam evaporation and this lays down in succession films of titanium 9 (an alternative metal for this layer would be chromium) having a thickness of 100nm, platinum 11 having thickness of 100nm and gold 12 having a thickness between 1000 and 3300nm (Figure 2B).

In order to enable a process of lift-off photolithography to be carried out, it was necessary to coat the metallised wafer with a resist material. This was done by prebaking the metallised wafer on a hotplate at 90°C for three minutes. Next, a positive resist material AZ4110 (Hoechst, FR Germany) was spread on the wafer and this was spun on a turntable at 4000 rpm for thirty seconds. The deposited resist was baked on a hotplate at 90°C for two minutes and then exposed to ultraviolet light at an intensity of $900Jm^{-2}$ in a photographic alignment machine. The exposure time was of the order of ten seconds. The substrate carrying the exposed layer was then soaked in chlorobenzene at 28° to 30°C for two minutes and blow dried. The layer was then developed in 'Microposit' developer (Hoechst, FR Germany) at 1:1 dilution in water and for a development time of sixty seconds. The resulting substrate carrying the developed layer was then rinsed carefully in water and blow dried. The resulting resist material 13 layer was of thickness 1100nm and it was provided with window openings at positions which had been determined by the photographic process (Figure 2C). The edges of the resist material at these openings will be seen to have overhanging edge regions and the particular shaping of these regions had been determined by the choice of resist material and the exposure/development times.

The provision of this resist stencil with overhang structure is necessary for the lift-off or float-off removal of unwanted metallised resist material which is required in the next step.

A further metallisation process was carried out and in this process, a thermal evaporation of silver caused a silver layer 14 of 400 to 1000nm thickness to be deposited over the whole of the resist layer and window structure (Figure 2D).

The resist is next removed by dissolution in acetone. This process leaves strips of silver only on the formerly unprotected parts of the metallised wafer surface (Figure 2E).

A process of wet chemical etching was then required to be carried out. This was done initially by spreading MPTS (3 -mercapto-propyl-trimethoxy-silane) liquid on the metallised wafer and spinning this on a turntable at 5000 rpm for forty seconds. This liquid acted as a surface adhesion promoter. Next, a positive resist material AZ4040 (Hoechst, FR Germany) was spread on the wafer and this was spun at 3000 rpm for sixty seconds. The wafer was then prebaked on a hotplate at 90°C for two minutes. It was next exposed to ultraviolet light at an intensity of 720Jm$^{-2}$ in a photographic alignment machine. The exposure time was about eight to ten seconds. The resulting layer was then developed in K400-AZ developer (Hoechst, FR Germany) at 1:4 dilution in water, for a development time of forty seconds. The resulting resist material layer was next postbaked on a hotplate at 90°C for two .minutes. This gave a layer of resist material 16 having a general thickness of 500nm (Figure 2F) but it will be noticed from the drawing that the resist layer additionally extends up the sides of the deposited silver layer 14 stripe and it partially covers the upper surface of the stripe.

A wet chemical etching of the silver was next able to be carried out and this was done using a ferric nitrate solution etchant. The resist material was subsequently removed by dissolution in acetone (Figure 2G).

A silver chloride deposit on the silver was next formed by making an electrical contact to the gold surface of the wafer and dipping this in a 1M hydrochloric acid solution. A constant current of ten microamperes per second per reference electrode pattern was then applied until a charge of 4 millicoulombs per pattern was reached. This created a silver chloride deposit in the form of a thin film on the silver suface.

A process of dry etching was then required to be carried out. This was done initially by spreading MPTS liquid on the metallised wafer and spinning this at 4000 rpm for sixty seconds. A positive resist material AZ4330 (Hoechst, FR Germany) was then spread on the wafer and this was spun at 3000 rpm for sixty seconds. The wafer was then prebaked on a hotplate at 90°C for five minutes. It was next exposed to ultraviolet light at an intensity of 2250Jm$^{-2}$ in a photographic alignment machine. The exposure time was about 23 to 25 seconds. The exposed layer was developed in K400-AZ developer at 1:3 dilution in water for sixty seconds.

The resulting resist layer was next postbaked on a hotplate at 90°C for fifteen minutes. It was then flow-baked on a hot plate at 110°C for five minutes. This stage caused the resist patterns to develop rounded edges and this feature was considered useful in order to obtain evenly etched metal patterns. The resulting resist material 17 layer was about 3500nm in thickness (Figure 2H).

An ion beam milling process was next carried out to etch away unwanted portions of gold, platinum and titanium so that the metallised areas were all provided as separate electrode regions on the electrically insulating silicon dioxide layer (Figure 2I).

As depicted in the drawing, an electrode area 18 capable of acting as an Ag/AgCl reference electrode is now seen to be located between two electrode areas 19 which would serve as working electrodes. After the silicon wafer 8 has been cut up into individual semiconductor chips, each chip can form the basis for a biosensor device construction. The portion of wafer depicted in Figure 2I will clearly be capable of being cut up to form two biosensor devices each having three electrodes.

The above explanation of the stages required for the construction of a biosensor device does not deal with the formation of the counter electrode structures which are located on the chip surface alongside the working and reference electrodes. It will be clear, however, that the counter electrodes can be formed by exactly the same process stages as those required for the smaller electrodes and suitable photographic masking should be able to provide these further electrodes without causing mechanical problems.

Some variations in this process have been devised and these are relevant to the treatment of the silver layer 14.

These variations are:-

(a) the silver chloride coating is capable of being formed after the ion beam milling stage. The coating may be formed electrolytically by making electrical contact to the silver electrodes.

(b) the silver may be chemically converted to silver chloride by treating with a freshly prepared solution of KCrO$_3$Cl (concentration 8 grams per litre) at 10nm/min as reported in the literature reference Sensors and Actuators, 9 (1986) 179-197 by L.J. Bousse, P. Bergveld and H.J.M Geeraedts.

(c) the silver may be deposited electrochemically on the pair of gold electrodes from a 3% silver plating solution (Silver Salt S30 from Johnson Matthey Chemicals). To do this, the electrodes were galvanostatically plated at the rate of one microampere per second until a total charge of 7 millicoulombs had been passed. The resulting electrodes had a shiny silvery appearance. The silver-plated electrodes were rinsed in distilled water and placed in a 1M hydrochloric acid solution for the electrolytic conversion to silver chloride, AgCl. Silver chloride was formed at a constant current of ten microamperes until a total charge of 4 millicoulombs had been passed. A dark brown deposit was observed on the electrode surface. Calibration of the prepared Ag/AgCl electrode against a standard Ag/AgCl reference electrode in a potassium phosphate buffer solution, at a pH value of 7, showed a maximum potential difference of 3mV. This confirmed that the microfabricated reference electrode of the present invention was comparable in output to that expected from the standard Ag/AgCl reference electrode.

The next step in the construction of the biosensor device is concerned with the immobilisation of the required enzyme catalyst material on the device.

## PREPARATION OF ENZYME ELECTRODES

Enzyme immobilization at the electrode surface was effected by entrapment of the enzyme material in an electrically conductive polypyrrole film, as follows:

### Example 1.

Immobilization of glucose oxidase at a 'smooth' textured gold electrode surface. The 'smooth' side of a semiconductor silicon wafer is defined as the side which is expected by the wafer manufacturer to be used for the construction of semiconductor devices. This surface has therefore been cleaned and polished so that it will be free of pits and roughness which would be likely to interfere with the device construction. There is a second side of the wafer which is normally left in an unfinished state by the wafer manufacturer after the drawn boule has been sawed to form the required wafers. This second side will be referred to as the 'rough' side of the wafer. It is not normally expected that this rough side will be used for the construction of semiconductor devices. However, in the construction of biosensor devices it has sometimes been found beneficial to form these on the wafer 'rough' side. To illustrate this effect, both the 'smooth' and the 'rough' textured wafer sides are used in these Examples.

The present Example is thus concerned with depositing the enzyme material on the 'smooth' wafer side. The enzyme immobilization was performed by the addition of 100U GOD (type VII from Aspergillus niger; Sigma Chemical Co) per cubic centimetre of solution to a deoxygenated solution of 0.1M phosphate buffer, pH 7, containing 0.1M potassium perchlorate and 0.1M pyrrole (Aldrich Chemical Co, redistilled once). The device was immersed in this polymerisation medium and enzyme film growth was initiated at one of the pairs of working electrodes by holding it at a constant potential of 0.8V against an Ag/AgCl reference electrode for approximately two hours. The total charge passed during film growth was approximately 250microcoulombs. The resulting enzyme electrode was rinsed with distilled water and it was then stored in a phosphate buffer solution at a temperature of 4°C for a period of at least one day before use.

### Example 2.

Immobilization of glucose oxidase at a 'rough' textured gold electrode surface. In this construction, the 'rough' side of the wafer was chosen for the metallisation and other stages of the fabrication process. The same procedure as for Example 1 was used.

### Example 3.

Immobilization of glucose oxidase at a platinum electrode. For preparing a platinum electrode, this was required to have a metal thickness of about 500 nanometres. To provide this, the gold from the upper surface of the metal patterns which consisted of the ternary metal coating of Ti/Pt/Au was removed by a dry or, alternatively, by a wet etching process. This was achieved by employing a suitable photolithographic mask, which protected the silicon surface and metal bonding pads but exposed only the serpentined metal patterns. The photolithographic process to obtain platinum was then carried out as explained in the aforementioned copending patent application.

For using the platinum electrode, the same protocol as for Example 1 was followed.

### Example 4.

Immobilization of glucose oxidase (GOD) at one pair of working electrodes and galactose oxidase (Ga0) at the other pair of working electrodes. GOD was codeposited with polypyrrole at one pair of electrodes, the device was then rinsed in distilled water and the second enzyme Ga0 in polypyrrole was immobilized at the other pair of working electrodes.

A series of electrochemical experiments was next carried out by using an EG & G Princeton Applied Research Potentiostat/Galvanostat Model 273 in conjunction with a JJ Instruments CR800 chart recorder. The biosensor cells for testing were mounted on a TO5 header package or alternatively on a ceramic chip carrier as used for packaging semiconductor devices. The biosensor cells (mounted on the TO5 header package) were temperature equilibrated to 25° ± 0.1°C by means of a glass water jacket connected to a Julabo F10 heating/circulating water bath. In the alternative system, if the biosensor chip is mounted on a ceramic chip carrier, it may be heated by a suitable Peltier block heater/cooler placed directly in intimate contact beneath the carrier and this is fitted in a specially made snap-on edge contact socket holder contained in a thermally insulated box. The socket holder is connected to miniature BNC sockets which are fitted on the outside of the box for making external coaxial connections. The temperature of the chip is controlled at 25° ± 0.1°C with the help of a thermistor fitted close to the chip and an external temperature control unit.

The potential between the sensing or working electrode and the reference electrode (Ag/AgCl) can be controlled at predetermined values, these values usually being programmable with time, by the use of the potentiostat. The control provided by this instrument can be effected regardless of any impedance changes which may occur in the electrochemical cell during the experiment.

The output steady state current is recorded, on the chart recorder, between the sensing or working electrode and the counter electrode. Those familiar with this measurement technique will be aware that the three electrode system can prevent the occurrence of any polarisation effects at the reference electrode.

**OPERATION OF THE ENZYME ELECTRODES AS AMPEROMETRIC SENSORS FOR THE ASSAY OF THEIR RESPECTIVE BIOLOGICAL SUBSTRATES, FOR EXAMPLE, SUGARS**

Glucose responses were measured by monitoring the oxidation of hydrogen peroxide with the working electrode poised at 0.7V against the Ag/AgCl reference electrode. The background current level was allowed to decay to a steady state before aliquot portions of stock glucose solution were added. Stock solutions of glucose in buffer had previously been prepared from a high purity β-D-glucose (British Drug House) material. After addition of glucose, the solution was agitated with a magnetic stirrer and then the passage of current in quiescent solution was recorded. A rapid increase in current flow was observed, a steady current state being reached within thirty seconds. The above method is employed when the sensor (contained in an open ended TO5 package) is dipped into the test solution. When the sensor is mounted on a ceramic chip carrier and is heated/cooled by a Peltier block arrangement, then the test solutions (sugar solutions) may be applied as droplets to the chip surface and no stirring of the solution is required. However, in this case, prepolarisation of the sensor chip in a buffer solution is required in order to get a rapid electrical response.

The assay of galactose at the galactose oxidase electrode was performed similarly with the electrode poised at 0.7V against the Ag/AgCl reference electrode and by measuring the steady state current response for a range of galactose concentrations.

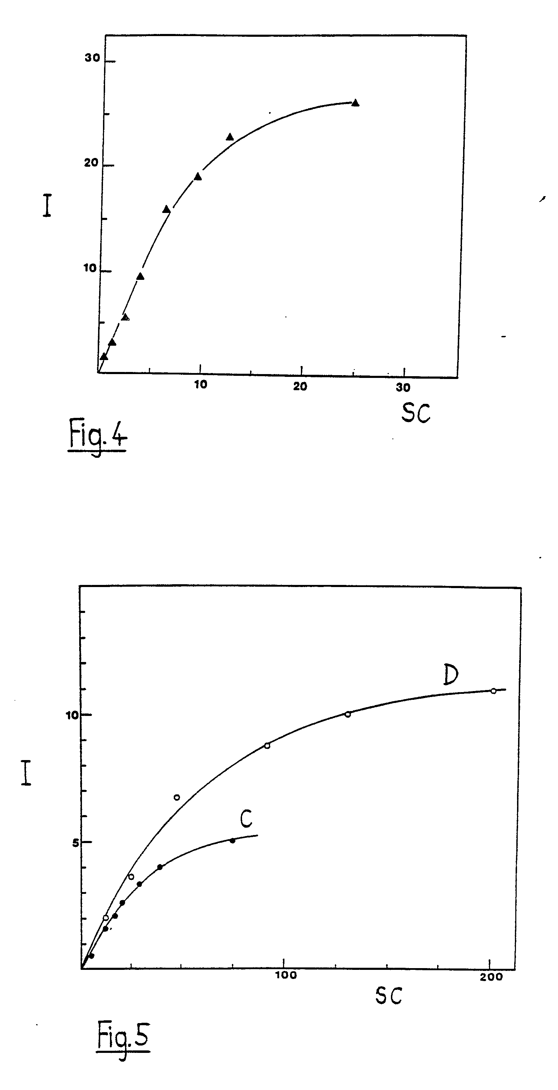

Figure 3 is a graph which shows the response curves for glucose at polypyrrole/glucose oxidase modified gold electrodes (Examples 1 and 2). The graph shows on the horizontal axis Glucose Concentration (SC), in millimoles, against current flow (1) in the steady state, measured in nanoampere units. The graph shows curves obtained by using both the 'smooth' (curve A) and 'rough' (curve B) textured gold electrodes. A larger current response was observed with the 'rough' gold electrodes indicating that amplification of the electrical signal had occurred as a result of an increase in the electrochemical surface area of the electrode material.

Figure 4 is another graph which shows the response curve for glucose at a polypyrrole/glucose oxidase modified platinum electrode (Example 3). The graph shows on the horizontal axis Glucose Concentration (SC), in millimoles, against current flow (I) in the steady state, measured in nanoampere units. It will be seen that for a Glucose Concentration up to about thirty millimoles the platinum electrodes give a substantially greater current flow than was evident with the gold electrodes of Figure 3.

Figure 5 is a further graph which shows the response curve of a dual enzyme sensor device for the respective enzyme substrates (Example 4), that is, for sugars. The graph shows on the horizontal axis Sugar Concentration (SC), in millimoles, against current flow (I) in the steady state, measured in nanoampere units. The dual enzyme sensor enables a response curve for glucose (curve C) to be plotted at the same time as a response curve for galactose (curve D). The use of this sensor can thus enable one of these sugars to be detected in the presence of the second sugar.

For convenience in use of the biosensor device, the silicon chip 2 may be mounted in one of the usual packages designed for semiconductor devices such as a TO5 header package or a ceramic chip carrier.

One advantage of the biosensor device of the present invention is that it is possible for the reference electrode to be built on a common chip with the working electrode(s). This can enable the voltage differential needed for the detection process to be kept at rather a low value, for example, 0.7 volts for sensing glucose and galactose. The result of this is to reduce possible electrical interference which will facilitate analysis for a single component in a mixture. If there should be other species present in the test sample, it would be possible for these other species to interfere if they could undergo chemical reaction for example in a range from above 0.7 volts to, say, 1.1 volts. This effect,if it was allowed to occur, would therefore be likely to considerably reduce the analytical sensitivity of the biosensor device.

Where the term biological catalyst is mentioned in the present specification, it will be clear that this material could be, for example, an enzyme, antibody, antigen or living cell material.

The foregoing description of an embodiment of the invention has been given by way of example only and a number of modifications may be made without departing from the scope of the invention ·as defined in the appended claims. For instance, it is not essential that the biosensor should be made of the very small dimensions described in the specific example, in a different embodiment the device could be made very much larger in size. This might be necessary if it was required for a range of different enzyme catalysts to be supported on the common substrate.

The electrode areas could of course be of any suitable shapes provided that the sensing electrode area is smaller that that of the counter electrode area, as would be dictated by the electrochemical requirements for this type of device. The present invention in fact provides two large counter electrode structures that can, if necessary, be used separately. This construction can thus allow two separate isolated biosensor devices to be used independently on the common substrate with the built-in reference electrode.

## Claims

1. A biosensor device comprising two spaced pairs of electrodes supported on a surface of electrical insulation material on a silicon substrate, one of said electrode pairs including a body of an immobilized reagent material incorporating an active biological catalyst which body is positioned between the electrodes of said pair to constitute a working electrode structure.

2. A device as claimed in Claim 1, in which the support surface additionally carries a third electrode pair to constitute a reference electrode structure.

3. A device as claimed in Claim 1 or 2, in which a second electrode pair also includes a body of an immobilized reagent material.

4. A device as claimed in Claim 1 or 2, in which a second electrode pair includes a body of an immobilized reagent material incorporating a second active biological catalyst.

5. A device as claimed in any one of Claims 1 to 4, in which the support surface additionally carries a further electrode having a surface area greater than that of said one electrode pair, the further electrode forming a counter electrode structure.

6. A device as claimed in any one of Claims 1 to 5, in which the electrodes are formed as thin film layers by an integrated circuit fabrication technique.

7. A device as claimed in any one of Claim 1 to 6, in which the said electrodes are formed of a noble metal such as gold or platinum.

8. A biosensor device, substantially as hereinbefore described with reference to Figure 1 of the accompanying drawings.

9. A method of fabricating a biosensor device as claimed in any one of Claims 1 to 8, in which the materials for forming an integral reference electrode are deposited by an electrolytic process.

10. A method of fabricating a biosensor device as claimed in any one of Claims 1 to 8, in which the biological catalyst material is deposited by an electrolytic process.

11. A method as claimed in Claim 9, in which the biological catalyst deposition is effected simultaneously with deposition of a polymer support film.

12. A method as claimed in Claim 9 or 10, in which the surface for the proposed biological catalyst deposition is initially masked with a non-electrically conductive material having a window formed therein, such that the deposit will only occur on a selected portion of the substrate.

13. A method as claimed in Claim 12, in which after deposition of a first biological catalyst material, the substrate is masked again and a second window is formed in the masking layer, the said second window serving for deposition of a second biological catalyst on a different portion of the

substrate.

Fig.1

Fig.3

Fig.2

Fig.4

Fig.5